# EUROPEAN PATENT APPLICATION

(11) **EP 3 406 186 A1**
(43) Date of publication of application: **28.11.2018**
(21) Application number: 17173010.4
(22) Date of filing: 26.05.2017
(51) Int. Cl.: A61B 5/00, A61B 5/055, A61B 5/06

(54) **A SENSOR CIRCUIT AND A SIGNAL ANALYZER FOR MEASURING AN IN-BODY PROPERTY**

(71) Applicant: Koninklijke Philips N.V., 5656 AE Eindhoven (NL)
(72) Inventor: DE WIJS, Willem-Jan Arend, 5656 AE Eindhoven (NL); DOODEMAN, Gerardus Johannes Nicolaas, 5656 AE Eindhoven (NL); KLEIJNEN, Mark, 5656 AE Eindhoven (NL)
(74) Representative: de Haan, Poul Erik

(57) **Abstract**

According to an aspect there is provided a sensor circuit for measuring an in body property, the sensor circuit comprising a resonant circuit that is responsive to a first radio frequency, RF, field to receive a carrier signal, the resonant circuit having: a first transducer, wherein a first electrical property of the first transducer is dependent on the in-body property, and a second transducer, wherein a second electrical property of the second transducer is dependent on a second pulsed field; wherein the carrier signal received by the resonant circuit is modulated by changes in the first electrical property due to the in-body property and changes in the second electrical property due to pulses in the second pulsed field.

## Description

### FIELD OF THE INVENTION

The invention relates to a sensor circuit and a signal analyzer for measuring an in-body property, and methods of operating the same.

### BACKGROUND OF THE INVENTION

In-body functional measurements are needed in minimally invasive procedures to complement imaging information. For example, devices known as guide wires can be placed into blood vessels using a minimally invasive procedure and guided through the blood vessels to a desired location, where some action or procedure is performed (e.g. placing a catheter or stent). In these procedures an imaging technique, such as ultrasound, x-ray or magnetic resonance imaging (MRI) is often used to provide images of the location of the guide wire in the body. When using these devices it can also be useful to obtain measurements of an in-body property, such as the pressure, temperature, conductivity, permeability and/or permittivity of the blood.

Current devices involve a wire leading from the sensor in the body to an external part of the device (i.e. a part of the device outside the body), necessitating alterations in the doctor's workflow to cope with the interconnection between sensor and external part of the device.

Currently proposed solutions to this problem entail wireless communication of an integrated handle of the device to which the wire is connected with a base station, which does not solve the problem, as each different device then needs an integrated device handle with at least two contacts (which then requires a correct alignment, and thus a complicated handle.

### SUMMARY OF THE INVENTION

It is an object of the present invention to provide an improved system, measurement (sensor) circuit and method for determining the value of an in-body property. It is also an object to provide an improved technique to enable the location of the measurement (sensor) circuit to be estimated.

The invention provides a system and method to mitigate the problems by using sensor circuit that can measure the property and enable the location of the measurement to be estimated.

According to a first aspect, there is provided a sensor circuit for measuring an in body property, the sensor circuit comprising a resonant circuit that is responsive to a first radio frequency, RF, field to receive a carrier signal, the resonant circuit having: a first transducer, wherein a first electrical property of the first transducer is dependent on the in-body property, and a second transducer, wherein a second electrical property of the second transducer is dependent on a second pulsed field; wherein the carrier signal received by the resonant circuit is modulated by changes in the first electrical property due to the in-body property and changes in the second electrical property due to pulses in the second pulsed field.

In some embodiments, the carrier signal received by the resonant circuit is modulated by changes in the first electrical property and changes in the second electrical property such that the modulated carrier signal comprises information on the in-body property and information on the pulses in the second pulsed field.

In some embodiments, the sensor circuit further comprises an output for outputting the modulated carrier signal to a signal analyzer.

In alternative embodiments, the resonant circuit is configured to transmit the modulated carrier signal to a signal analyzer.

In some embodiments, the first electrical property of the first transducer is the capacitance, inductance and/or resistance of the first transducer.

In some embodiments, the second electrical property of the second transducer is the capacitance, inductance and/or resistance of the second transducer.

In some embodiments, the second transducer comprises a first transducer component that generates a voltage responsive to the second pulsed field; and a second transducer component coupled to the first transducer component, the second transducer component having the second electrical property, and wherein the second transducer component is such that the second electrical property is dependent on the voltage generated by the first transducer component.

In some embodiments, the second transducer component has a non-linear relation between voltage and current in amplitude and phase.

In some embodiments, the second transducer component is such that the first transducer and the second transducer component generates at least one pair of sidebands for the modulated carrier signal during a first phase of the second pulsed signal.

In some embodiments, the second transducer component is a diode or a varactor diode.

In some embodiments, the resonant circuit further comprises a third transducer, wherein a third electrical property of the third transducer is dependent on the in-body property, and wherein the third transducer is spaced from the first transducer such that the first transducer and the third transducer measure the in-body property at different positions in a body; wherein the first transducer and the second transducer component form a first resonant sub-circuit; and wherein the second transducer further comprises: a third transducer component coupled to the first transducer component, and coupled to the third transducer to form a second resonant sub-circuit, the third transducer component having the second electrical property, and wherein the third transducer component is such that the second electrical property is dependent on the voltage generated by the first transducer component.

In some embodiments, the second transducer component and the third transducer component are arranged such that the first transducer and the second transducer component generate at least one pair of sidebands for the modulated carrier signal during a first phase of the second pulsed signal, and such that the third transducer component and the third transducer generate at least one pair of sidebands for the modulated carrier signal during a second phase of the second pulsed signal, wherein the second phase is opposite to the first phase.

In some embodiments, the second non-linear component is a diode or a varactor diode.

In some embodiments, the in-body property is any one or more of pressure, temperature, conductivity, permeability and permittivity.

In some embodiments, the second pulsed field is an ultrasound field, an X-ray field, an electromagnetic, EM, field used in magnetic resonance imaging, MRI, or light.

According to a second aspect, there is provided a signal analyzer for determining a measurement of an in-body property, the signal analyzer comprising a processing unit configured to receive a first modulated carrier signal generated by a sensor circuit; receive first information regarding a first radio, RF, field that the sensor circuit has been exposed to; receive second information regarding a second pulsed field that the sensor circuit has been exposed to; and analyze the received first modulated carrier signal using the received first information and the received second information to determine a measurement of the in-body property.

In some embodiments, the processing unit is configured to analyze the received first modulated carrier signal by comparing amplitude, phase and/or frequency of the first RF signal to the amplitude, phase and/or frequency of the received first modulated carrier signal to determine a measurement of the in-body property.

In some embodiments, the processing unit is configured to analyze the received first modulated carrier signal by: comparing amplitude, phase and/or frequency of pulses in the second pulsed signal to the received first modulated carrier signal to determine a timing of the modulated carrier signal.

In some embodiments, the processing unit is configured to analyze one or more sidebands of the received first modulated carrier signal according to a phase of pulses in the second pulsed field.

In some embodiments, the processing unit is configured to analyze a first pair of sidebands of the received first modulated carrier signal according to a first phase of pulses in the second pulsed field to determine a measurement of the in-body property by a first transducer, wherein a first electrical property of the first transducer is dependent on the in-body property; and analyze a second pair of sidebands of the received first modulated carrier signal according to a second phase of pulses in the second pulsed field to determine a measurement of the in-body property by a second transducer, wherein the second phase is opposite to the first phase and wherein a second electrical property of the second transducer is dependent on the in-body property.

In some embodiments, the processing unit is further configured to compare amplitude, phase and/or frequency of pulses in the second pulsed signal to the received first modulated carrier signal to determine a timing of the received modulated carrier signal; estimate an angle and/or direction from which the first modulated carrier signal was received; and estimate a position of the sensor circuit based on the timing of the first modulated carrier signal and the estimated angle and/or direction.

In some embodiments, the processing unit is further configured to receive at least a second modulated carrier signal generated by the sensor circuit, wherein the first modulated carrier signal and the second modulated carrier signal are received at different positions with respect to the sensor circuit; compare amplitude, phase and/or frequency of pulses in the second pulsed signal to the received first modulated carrier signal to determine a timing of the first modulated carrier signal; compare amplitude, phase and/or frequency of pulses in the second pulsed signal to the received second modulated carrier signal to determine a timing of the second modulated carrier signal; and estimate a position of the sensor circuit based on the timing of the first modulated carrier signal and the timing of the second modulated carrier signal.

In some embodiments, the signal analyzer further comprises one or both of a first field generator for generating the first RF field; and a second pulsed field generator for generating the second pulsed field.

According to a third aspect, there is provided a system for measuring an in-body property comprising a sensor circuit as described above; and a signal analyzer as described above

In some embodiments, the system further comprises one or both of a first field generator for generating the first RF field; and a second pulsed field generator for generating the second pulsed field.

According to a fourth aspect, there is provided a method of operating a sensor circuit to measure an in-body property, the method comprising receiving a carrier signal in a resonant circuit, the resonant circuit being responsive to a first radio frequency, RF, field, the resonant circuit comprising a first transducer and a second transducer, wherein a first electrical property of the first transducer is dependent on the in-body property, and a second electrical property of the second transducer is dependent on a second pulsed field; responsive to the second pulsed field, modulating the received carrier signal to form a modulated carrier signal, wherein the received carrier signal is modulated by changes in the first electrical property and changes in the second electrical property such that the modulated carrier signal comprises information on the in-body property and information on pulses in the second pulsed field.

In some embodiments, the step of modulating comprises modulating the carrier signal received by the resonant circuit with changes in the first electrical property and changes in the second electrical property such that the modulated carrier signal comprises information on the in-body property and information on the pulses in the second pulsed field.

In some embodiments, the method further comprises outputting the modulated carrier signal to a signal analyzer. In some embodiments, the step of outputting comprises transmitting the modulated carrier signal to a signal analyzer.

In some embodiments, the first electrical property of the first transducer is the capacitance, inductance and/or resistance of the first transducer.

In some embodiments, the second electrical property of the second transducer is the capacitance, inductance and/or resistance of the second transducer.

In some embodiments, the second transducer comprises a first transducer component that generates a voltage responsive to the second pulsed field; and a second transducer component coupled to the first transducer component, the second transducer component having the second electrical property, and wherein the second transducer component is such that the second electrical property is dependent on the voltage generated by the first transducer component.

In some embodiments, the second transducer component has a non-linear relation between voltage and current in amplitude and phase.

In some embodiments, the second transducer component is such that the first transducer and the second transducer component generates at least one pair of sidebands for the modulated carrier signal during a first phase of the second pulsed signal.

In some embodiments, the second transducer component is a diode or a varactor diode.

In some embodiments, the resonant circuit further comprises a third transducer, wherein a third electrical property of the third transducer is dependent on the in-body property, and wherein the third transducer is spaced from the first transducer such that the first transducer and the third transducer measure the in-body property at different positions in a body; wherein the first transducer and the second transducer component form a first resonant sub-circuit; and wherein the second transducer further comprises: a third transducer component coupled to the first transducer component, and coupled to the third transducer to form a second resonant sub-circuit, the third transducer component having the second electrical property, and wherein the third transducer component is such that the second electrical property is dependent on the voltage generated by the first transducer component.

In some embodiments, the second transducer component and the third transducer component are arranged such that the first transducer and the second transducer component generate at least one pair of sidebands for the modulated carrier signal during a first phase of the second pulsed signal, and such that the third transducer component and the third transducer generate at least one pair of sidebands for the modulated carrier signal during a second phase of the second pulsed signal, wherein the second phase is opposite to the first phase.

In some embodiments, the second non-linear component is a diode or a varactor diode.

In some embodiments, the in-body property is any one or more of pressure, temperature, conductivity, permeability and permittivity.

In some embodiments, the second pulsed field is an ultrasound field, an X-ray field, an electromagnetic, EM, field used in magnetic resonance imaging, MRI, or light.

According to a fifth aspect, there is provided a method for measuring an in-body property using a sensor circuit, the method comprising receiving a first modulated carrier signal generated by the sensor circuit; receiving first information regarding a first radio frequency, RF, field that the sensor circuit has been exposed to; receiving second information regarding a second pulsed field that the sensor circuit has been exposed to; and analyzing the received first modulated carrier signal using the received first information and the received second information to determine a measurement of the in-body property.

In some embodiments, the step of analyzing comprises analyzing the received first modulated carrier signal by comparing amplitude, phase and/or frequency of the first RF signal to the amplitude, phase and/or frequency of the received first modulated carrier signal to determine a measurement of the in-body property.

In some embodiments, the step of analyzing comprises analyzing the received first modulated carrier signal by: comparing amplitude, phase and/or frequency of pulses in the second pulsed signal to the received first modulated carrier signal to determine a timing of the modulated carrier signal.

In some embodiments, the step of analyzing comprises analyzing one or more sidebands of the received first modulated carrier signal according to a phase of pulses in the second pulsed field.

In some embodiments, the step of analyzing comprises analyzing a first pair of sidebands of the received first modulated carrier signal according to a first phase of pulses in the second pulsed field to determine a measurement of the in-body property by a first transducer, wherein a first electrical property of the first transducer is dependent on the in-body property; and analyzing a second pair of sidebands of the received first modulated carrier signal according to a second phase of pulses in the second pulsed field to determine a measurement of the in-body property by a second transducer, wherein the second phase is opposite to the first phase and wherein a second electrical property of the second transducer is dependent on the in-body property.

In some embodiments, the method further comprises the step of comparing the amplitude, phase and/or frequency of pulses in the second pulsed signal to the received first modulated carrier signal to determine a timing of the received modulated carrier signal; estimating an angle and/or direction from which the first modulated carrier signal was received; and estimating a position of the sensor circuit based on the timing of the first modulated carrier signal and the estimated angle and/or direction.

In some embodiments, the method further comprises receiving at least a second modulated carrier signal generated by the sensor circuit, wherein the first modulated carrier signal and the second modulated carrier signal are received at different positions with respect to the sensor circuit; comparing amplitude, phase and/or frequency of pulses in the second pulsed signal to the received first modulated carrier signal to determine a timing of the first modulated carrier signal; comparing amplitude, phase and/or frequency of pulses in the second pulsed signal to the received second modulated carrier signal to determine a timing of the second modulated carrier signal; and estimating a position of the sensor circuit based on the timing of the first modulated carrier signal and the timing of the second modulated carrier signal.

In some embodiments, the method further comprises one or both of generating the first RF field; and generating the second pulsed field.
In a further aspect of the present invention a sensor circuit for measuring an in body property is provided, comprising: a first transducer by modulating a first field to which the first transducer is exposed, and a second transducer for modulating a second pulsed field to allow timing information of the sensor circuit.

In another further aspect of the present invention a system for measuring an in body property is provided, comprising: the above sensor circuit, a first field generator for generating a first field to expose the sensor circuit, a second field generator for generating a second pulsed field to expose the sensor circuit, a first determination unit for determining the measurement of the property, and a second determination unit for determining the position of the sensor circuit.

According to an aspect of the invention the position of a sensor is wirelessly transferred using RF field modulation, using the timing information provided by the externally applied ultrasound (US) pulse from an US imaging probe.

By including a sensor in the resonant coil that also changes capacitance as a function of the property (parameter) to measure (for instance an air capacitor to measure pressure), the possibility exists to measure this second sensor property as well using the RF field. In an embodiment, in each phase of the externally applied pulse a different coil can be detuned, enabling differential measurements with one common externally applied pulse.

Instead of a pulsed US field, a pulsed X-ray or MRI pulse can be used, with the appropriate transducer to convert the energy of the field to electrical capacitance.

The change in resonance (detuning) of the multiple coils can be detected either wirelessly using one or more RF receivers, or it can be transmitted over a wire to a detector unit (which is also referred to herein as a signal analyzer).

Thus, the problem of interconnection is overcome by the invention. In a wireless embodiment, elimination of wires to transfer power and data to and from the sensor circuit results in a so-called 'freedom wire' that allows the physician to operate the guide wire with full freedom (i.e. without having to consider how to measure the in-body property). In the 'one wire embodiment', the sensor circuit according to the invention can use a simple clamp or connection onto the ground wire to interconnect the sensor circuit to the external measurement box.

The data transfer over an RF carrier wave also enables a plurality of sensor assemblies to be connected on the same (guide) wire, as every sensor assembly can be tuned to respond to a specific RF frequency carrier signal. This enables pressure wave propagation speed measurements (or other in-body property measurements) on multiple parts of the guide wire, which is beneficial in FFR (fractional flow reserve) measurements, as no pull back of the wire is needed when the occlusion is passed, as there is always a sensor assembly on the wire behind the occlusion.

When the RF signal detuning is not just measured with one RF receiver but wirelessly with multiple (at least three), the position of the sensor can also be determined by 'triangulation', allowing position determination relative to the reference system of the RF receivers. The RF receiver reference system can then be coupled to the patient reference system.

The external pulsed field energy needed can be provided in multiple ways, with MRI type radiation, X-ray and US radiation. The benefit of MRI and X-Ray is that the field can be applied into the body from a fairly large distance, whereas in the case of ultrasound the field needs to be generated by a device in contact with the body, for instance a wearable patch or an ultrasound probe. Also light can be used as external pulse source, if the sensor circuit is not too deep inside the body or the light source is also inserted into the body.

These and other aspects of the invention will be apparent from and elucidated with reference to the embodiments described hereinafter.

### BRIEF DESCRIPTION OF THE DRAWING

For a better understanding of the invention, and to show more clearly how it may be carried into effect, reference will now be made, by way of example only, to the accompanying drawings, in which:
Fig. 1 shows a block diagram of the system according to the invention,
Fig. 2 shows a block diagram of a sensor circuit according to the invention,
Fig. 3 shows a schematic of the sensor circuit for a dual transducer assembly according to an embodiment of the invention,
Fig. 4 schematically shows the pressure wave propagation measured with two transducers according to an embodiment of the invention,
Fig. 5 is a functional representation of how a sensor circuit can be operated to produce a modulated carrier signal according to an embodiment of the invention,
Fig. 6 shows a block diagram of a signal analyzer according to the invention,
Fig. 7 shows a flow chart of a method of operating a sensor circuit according to an embodiment of the invention,
Fig. 8 shows a flow chart of a method for measuring an in-body property using a sensor circuit according to an embodiment of the invention,
Fig. 9 schematically shows a side view of a wireless design of the sensor circuit according to an embodiment of the invention,
Fig. 10 schematically shows mounting a sensor circuit inside a shrink tube.

### DETAILED DESCRIPTION OF EMBODIMENTS

The invention is based on the insight that frequency and phase information from an external pulsed field can be used to 'lock in' the timing of the read-out of data that has a low amplitude compared to external applied RF field and noise. The detection involves algorithms and software to extract the data from the raw signal.

This understanding enriches the number of possible applications and the quality of the data collected with these applications. Although the invention is described with reference to a guide wire, it will be appreciated that the invention is not limited to this usage, and, for example, the invention can be implemented as a standalone implantable device that is to measure an in-body property. The in-body property could be any of pressure, a pressure velocity field, flow velocity, temperature, conductivity, permeability and permittivity of a body part in contact with the device, and for example where the device is used within a blood vessel, such as a vein or artery, the in-body property can be any one or more of blood pressure, the pressure velocity field in the blood, the blood flow velocity, temperature of the blood, conductivity of the blood, permeability of the blood and permittivity of the blood.

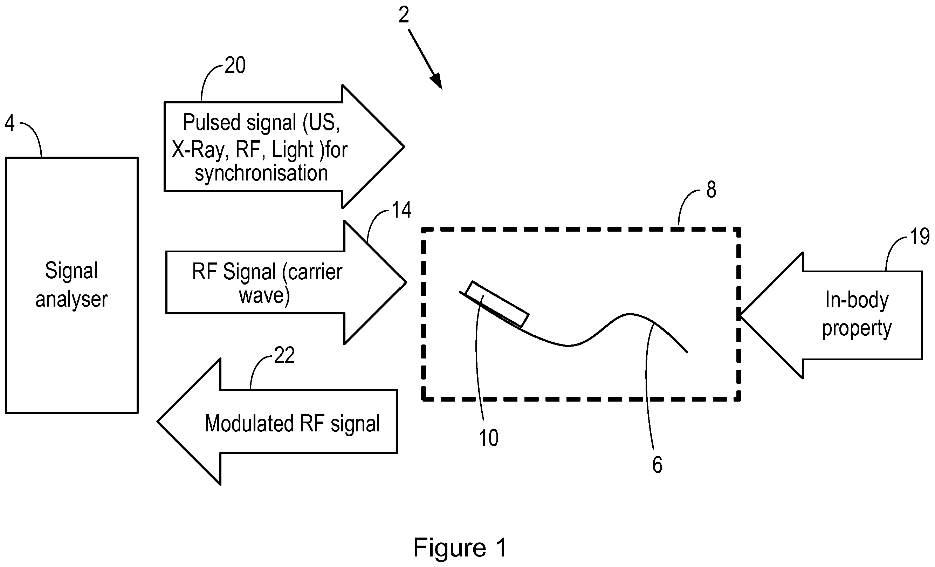

A block diagram of an exemplary system 2 is given in Figure 1. Figure 1 shows a signal analyzer 4 (which is also referred to as an RF wireless sensing unit) which in some embodiments causes modulation/timing synchronization according to the invention using an additional external pulsed field. The signal analyzer 4 determines the measurement of the in-body parameter from a received signal. Also shown in Figure 1 is a guide wire 6 that can be used inside a body of a patient or subject 8, with the guide wire 6 having a sensor circuit 10 according to the invention.

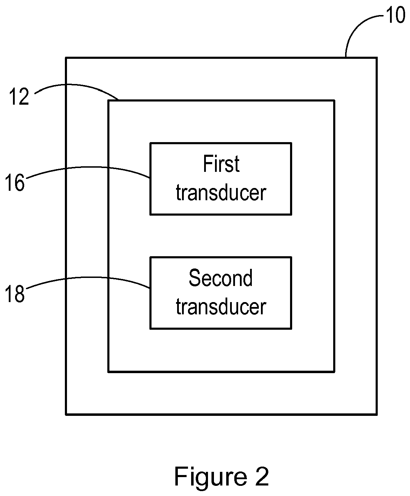

Figure 2 is a block diagram of a sensor circuit 10 according to an embodiment of the invention. The sensor circuit 10 comprises a resonant circuit 12 that is responsive to a radio frequency (RF) field to receive a carrier signal. The resonant circuit 12 comprises a first transducer 16 and a second transducer 18. The RF field can be generated by the signal analyzer 4, as shown by arrow 14 in Figure 1, although it will be appreciated that alternatively the RF field can be generated by a separate device to the signal analyzer 4.

The first transducer 16 acts to measure the in-body property of interest (e.g. pressure, flow velocity, etc.). In particular, the first transducer 16 is such that an electrical property of the first transducer 16 is dependent on the in-body property to be measured. The 'influence' of the in-body property is shown by arrow 19. For example the electrical property can be the capacitance, inductance and/or resistance of the first transducer 16. In a specific example, as described in more detail below, the first transducer 16 is an air capacitor (i.e. a capacitor that uses air as the dielectric), and the first transducer 16 is arranged on the sensor circuit 10 such that it is exposed to the blood of the subject, and the blood pressure affects the spacing of the plates in the capacitor, and thus affects the capacitance. The capacitance of the capacitor is therefore dependent on the blood pressure.

The second transducer 18 is provided to respond to a pulsed signal having known timing properties (i.e. the timing of the pulse is known) in order to modulate the received carrier signal, and thus 'encode' timing information into the received carrier signal (since the measurements are read out at a time corresponding to a pulse). Thus the second transducer 18 is such that an electrical property of the second transducer 18 is dependent on a pulsed field, in particular a pulse in the pulsed field.

The pulsed field can be generated by the signal analyzer 4, as shown by arrow 20 in Figure 1, although it will be appreciated that the pulsed field can be generated by a separate device to the signal analyzer 4, and/or to the unit that generated the RF field. The pulsed field 20 maybe another RF field, but the pulsed field 20 can alternatively be an ultrasound (US) field, an X-Ray field, an electromagnetic (EM) field used in MR imaging, or light.

The characteristics of the pulsed field are such that the pulse has a sufficiently short duration to allow a defined measurement time to be identified. The pulse repetition rate is mainly needed to allow multiple measurements in a short time. The pulsed signals in the pulsed field can be in a frequency range of about 0%-5% of the RF resonance frequency of the resonant circuit 12. Some embodiments of the pulsed field are described below, but it will be appreciated that these are merely exemplary, and those skilled in the art will be aware that pulsed fields having different characteristics can be used.

For ultrasound pulsed fields the following ranges can be used: a frequency of 1-20 MHz frequency, with a pulse width between 0.1 and 10 µs, and a pulse repetition frequency between 1 kHz to 10 kHz. An embodiment of the sensor circuit 10 that can be used with an ultrasound pulsed field is described in more detail below.

For a light-based pulsed field, light emitting diode (LED) pulses (flashes) of 100Hz to 500 Hz can be used with a pulse width of typically between 0.1 and 10 µs to obtain well defined measurement time points. In this embodiment the second transducer 18 can be a light sensitive diode.

For an X-Ray pulsed field, a mechanical shutter can be used in order to create well-defined short X-Ray pulses, for example with a pulse width of 10-20 µs, with a pulse repetition rate of 100-200 Hz. In this embodiment the second transducer 18 can be a material that responds to X-rays.

In some embodiments, the second transducer 18 comprises a first transducer component that converts the pulsed field 20 into a positive and negative charge (and so the first transducer component acts like a charge source when exposed to the ultrasound field), and a non-linear component that has a non-linear relation between voltage and current in amplitude and phase. The non-linear component is referred to herein as a second transducer component. In some embodiments, the second transducer component can be such that its capacitance is dependent on an applied voltage. In this case the electrical property of the second transducer is capacitance. In some embodiments, the second transducer component is a variable capacitance diode, which is also known as a varactor or a varactor diode. In other embodiments the second transducer component can be an inductor with a magnetic core material close to magnetic saturation, and this inductor can translate the applied voltage from the first transducer component into a variable non-linear behavior.

In the example below in which the pulsed field 20 is an ultrasound field, the first transducer component can be a piezoelectric transducer (e.g. a transducer formed from polyvinylidene fluoride (PVDF)), and the second transducer can be a varactor.

In embodiments where an alternative pulsed field 20 is used (e.g. an X-Ray, MRI or light field), the first transducer component can be a component that responds to that pulsed field (e.g. to produce a voltage in response to the pulses), and the second transducer component can be a varactor.

The first transducer 16 and second transducer 18 are arranged in the resonant circuit 12 so that the carrier signal 14 received by the resonant circuit is modulated by changes in the electrical property of the first transducer 16 due to the in-body property and changes in the electrical property of the second transducer 18 due to pulses in the pulsed field. More specifically, the changes in the electrical properties of the first transducer 16 and the second transducer 18 change the resonance of the RF antenna formed by the resonant circuit 12, thereby modulating the received RF carrier signal with both the measurement of the in-body property and timing information relating to the pulses in the pulsed field.

The modulated RF carrier signal (shown by arrow 22 is output by the sensor circuit 10 to the signal analyzer 4. In some embodiments, the sensor circuit 10 comprises a wire or wires for outputting the modulated carrier signal 22 to the signal analyzer. In other embodiments, the sensor circuit 10 is configured to transmit the modulated carrier signal 22 to a signal analyzer. In these embodiments, the sensor circuit 10 can comprise a powered transmitter and antenna (although in that case the sensor circuit 10 also requires a power source, e.g. a battery), or a passive antenna that transmits the modulated RF carrier signal 22 (similar to a passive radio frequency ID (RFID) tag).

In some embodiments, which are described in more detail below, the sensor circuit 10 can be used to measure the in-body property at two or more positions in the body. Thus, the sensor circuit 10 can comprise one or more additional transducers (referred to herein as a third transducer), similar to the first transducer 16, i.e. a transducer that is such that an electrical property of the transducer is dependent on the in-body property to be measured. When the sensor circuit 10 is used on a guidewire, the first transducer 16 and the third transducer can be at different positions along the guidewire so as to measure the in-body property at different positions in the body.

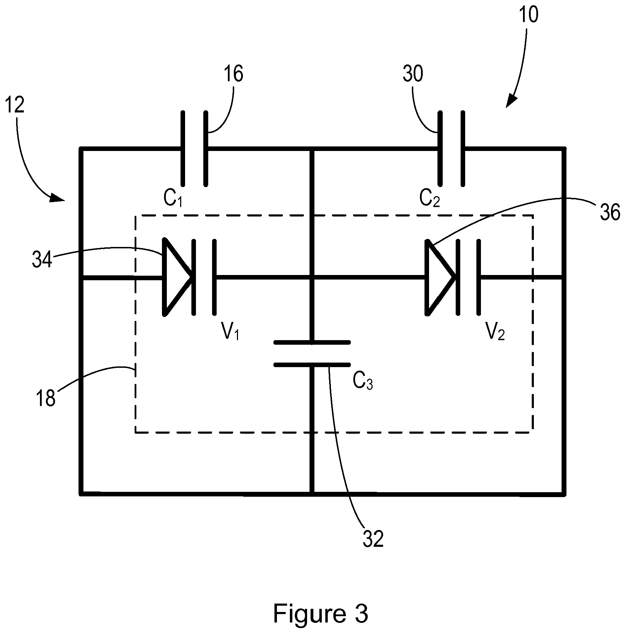

Figure 3 shows an exemplary simplified circuit diagram of a sensor circuit 10 that can measure the in-body property at two different locations in the body according to an embodiment. In particular, in this embodiment the in-body property is pressure, and thus the sensor circuit 10 comprises a first transducer 16 in the form of an air capacitor (C₁), a second transducer 18 as described below that converts external pulsed energy into a positive and negative charge (i.e. the second transducer 18 acts as a charge source in the external pulsed field), and a third transducer 30 in the form of an air capacitor (C₂). Those skilled in the art will appreciate that the sensor circuit 10 can be readily adapted to measure alternative in-body parameters by substituting the air capacitors for suitable transducers that are responsive to the in-body property of interest, and the explanation of Figure 3 below should not be considered as limited to measuring pressure.

The second transducer 18 comprises a first transducer component 32 (C₃) that is responsive to the pulsed field 20 (e.g. ultrasound) to generate a voltage in accordance with the pulses, a second transducer component 34 (V₁) that forms a first resonant sub-circuit with the first transducer 16 and a third transducer component 36 (V₂) that forms a second resonant sub-circuit with the second transducer 18. The first transducer component 32 can be in the form of a piezoelectric transducer. The second transducer component 34 (V₁) and the third transducer component 36 (V₂) can be varactors. The second transducer component 34 and the third transducer component 36 are arranged such that the first transducer 16 and the second transducer component 34 generate at least one pair of sidebands for the modulated carrier signal 22 during a first phase of the pulsed signal (e.g. a positive phase), and such that the third transducer component 36 and the third transducer 30 generate at least one pair of sidebands for the modulated carrier signal 22 during a second phase of the pulsed signal (e.g. a negative phase). The positive phase of the external pulse results in de-tuning of coil C₁-V₁ (the first resonant sub-circuit) due to a change in capacitance of V₁, the negative phase of the external pulse results in de-tuning of coil C₂-V₂ (the second resonant sub-circuit) due to a change in capacitance of V₂, allowing the two different coils to be read out at the same time in different phases of the pulsed field. The difference in the in-body property between pressure sensor C₁ and C₂ results in a different tuning value for the two coils (resonant subcircuits). When these pressure values are tracked in time, the propagation speed of the pressure wave can be calculated using the physical distance dX between C₁ and C₂. Preferably the detuning effect of V₁ and V₂ is about 2-10 times greater than the de-tuning effect of C₁ and C₂ to allow accurately timed measurements.

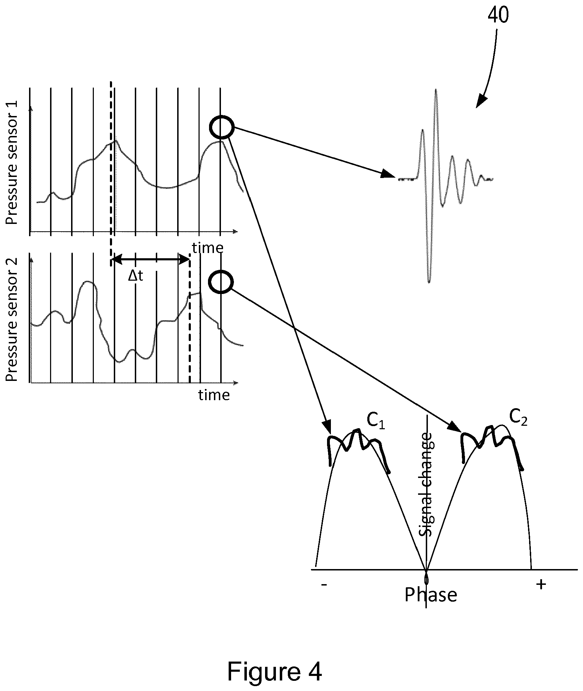

As noted above, one embodiment or use of the sensor circuit 10 in Figure 3 is to measure flow from differential pressure of two sensors (i.e. the transducers 16, 30 that measure the in-body property), where it is not the absolute value of the pressure difference that is important, but the phase shift between the data of the two sensors (i.e. the two transducers 16, 30). This circumvents the drift issues of existing solutions. A conceptual data structure for pulsed external field and signal from two pressure-sensitive transducers is given in Figure 4.

Figure 4 shows pressure wave propagation measured with two sensors/transducers 16, 30 spaced a known distance dX apart (with the first transducer 16 upstream of the third transducer 30 so that a pressure pulse reaches the first transducer 16 before reaching the third transducer 30), and the same pressure (i.e. a blood pulse) is measured by each transducer at an interval dT. The interval dT is determined accurately by the timing of the externally applied pulsed field 20, and thus the pulse velocity can be determined from dX/dT.

In the top right part of Figure 4 an example of the external pulse 40 of the pulsed field 20 is given, where it can be clearly seen that positive and negative phases are present in the pulse 40. Each vertical line in the pressure sensor graphs on the left hand side corresponds to a single pulse 40 in the pulsed field. Each pulse 40 is linked to the read out of measurements of pressure by both transducers 16, 30. In other words, the measurements of the pressure is read out of the first transducer 16 and the third transducer 30 each time that a pulse 40 is applied. The pressure measurement by pressure sensor 1 (the first transducer 16) is read out in the negative phase of the pulse 40, and the pressure measurement by pressure sensor 2 (the third transducer 30) is read out in the positive phase of the pulse 40, as shown by the graph in the bottom right corner.

Figure 4 also shows a graph illustrating how the sideband signal change is clearly linked to the phase of the pulse, which means that pulse timing information (i.e. information on the timing and/or phase of the pulse) is contained in the modulated carrier signal. Each varactor (or other non-linear element) causes amplitude and phase modulation on the RF carrier which appears as lower and upper side bands. The varactors (or other non-linear element) react asymmetrically to the applied voltage, so the upper and lower sidebands are unequal. Thus, the change in the electrical property of the first transducer 16 and the change in the electrical property of the third transducer 30 will be phase separated in the modulated carrier signal 22. This means that a pressure rise (or decrease) on the two transducers 16, 30 can be recognized independently.

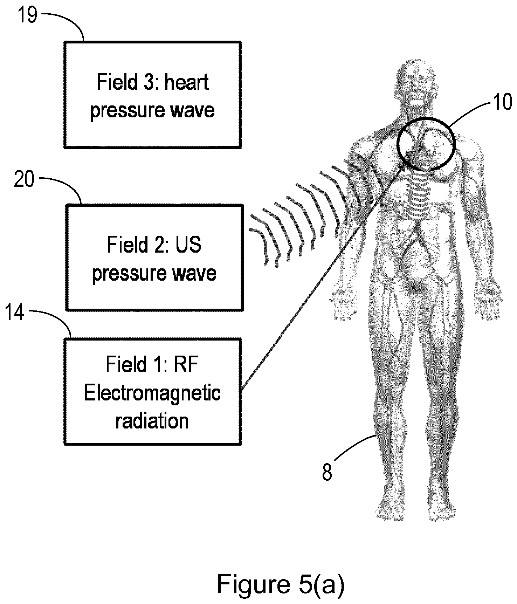

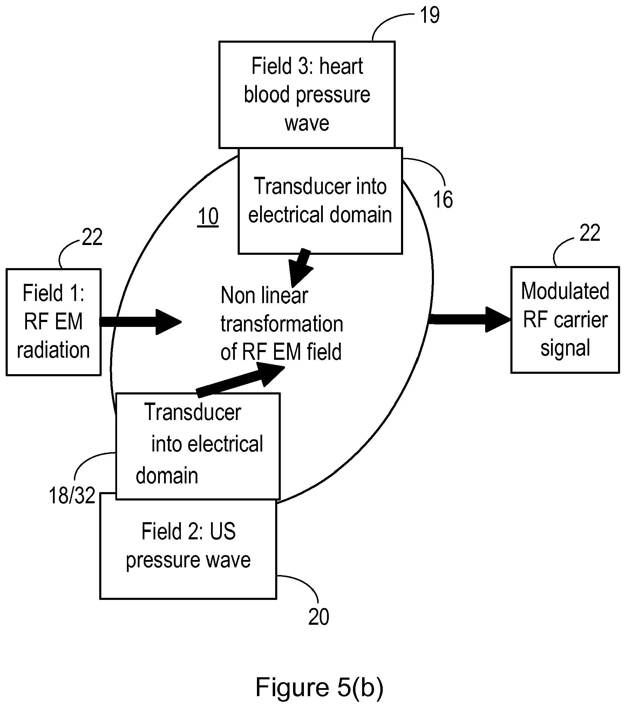

Figure 5 is a functional representation of how a sensor circuit 10 can be operated to produce a modulated carrier signal 22 according to an embodiment of the invention. In particular Figure 5 shows how the RF carrier signal 14, pulsed signal 20 and the measurement of the in-body property 19 are 'combined' to produce the modulated carrier signal 22. Figure 5(a) shows a subject 8 that has a sensor circuit 10 located inside their body (e.g. inside a blood vessel) to measure pressure in the blood (e.g. a heart pressure wave 19). The RF field 14 is shown, along with an ultrasound pulsed pressure field 20 and a 'field' 19 representing the heart pressure wave.

Figure 5(b) shows the sensor circuit 10 with the first transducer 16 that converts changes in the blood pressure wave 19 into the electrical domain, the second transducer 18 that converts the ultrasound pulses into the electrical domain, which both 'combine' to effect a non-linear transformation of the RF carrier signal 14 to generate the modulated carrier signal 22.

The RF field 14 can penetrate the human body and so reach the sensor circuit 10 inside the body 8. A suitable frequency for the RF field 14 is in the 400-430 MHz range (although other frequencies can be used), as a field at this frequency has a penetration depth of greater than 30 cm.

In the absence of any other changes to the RF signal 14 (i.e. without the influence of the first and second transducers 16, 30), the reflection of the RF field 14 on a sensor circuit can be measured, in terms of intensity and/or phase, for example. However, without further transformation of the RF field 14, the reflections are hard to detect. The normal principles of RFID are to modulate the RF field (linearly) with a stored code or a transmitted code (e.g. a mechanical vibration, an optical pulse, etc.).

As noted above, the invention provides a non-linear element (or elements) that transduces properties (e.g. a measurement of the in-body property and timing information) into the RF field 14 by a pulsed field 20 (e.g. an ultrasound field) and transduces the in-body property measurements into the RF field 14.

In some embodiments, the ultrasound pressure waves are transduced by a PVDF foil (or other form factor) into a voltage. The varactor is the non-linear element that changes the capacitance of the RF tuned circuit (the resonant circuit 12), but only if reverse bias is applied, so only in one phase of the pulse (e.g. the positive phase). This generates side-bands in the RF signal of different frequencies that can be detected in the signal analyzer by filtering the main RF frequency out of the modulated carrier signal 22. The timing information contained in the modulated carrier signal 22 from the pulsed field has a very characteristic frequency spectrum that is known from the ultrasound transducer used to generate the pulsed field. The use of two different varactors means that each can modulate the RF carrier signal 14 during a respective phase of the pulsed field (i.e. positive or negative).

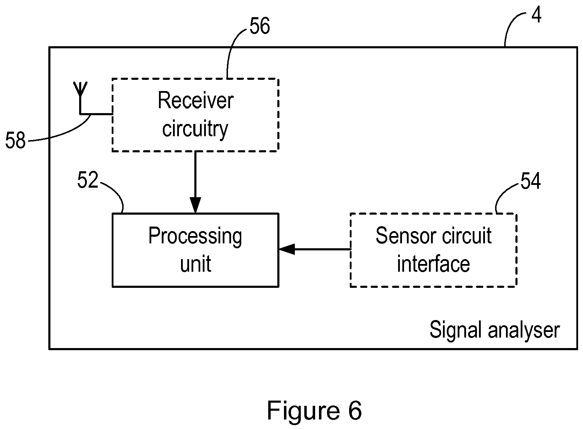

Figure 6 is a block diagram of a signal analyzer 4 according to an embodiment. The signal analyzer 4 comprises a processing unit 52 that generally controls the operation of the signal analyzer 4 and that determines the measurement of the in-body property and/or the timing information relating to the modulated RF signal 22.

The processing unit 52 can be implemented in numerous ways, with software and/or hardware, to perform the various functions described below. The processing unit 52 may comprise one or more microprocessors or digital signal processors (DSPs) that may be programmed, using software or computer program code, to perform the required functions and/or to control components of the processing unit 52 to effect the required functions. The processing unit 52 maybe implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 52 can comprise or be associated with a memory unit (not shown in Figure 6), such as a volatile or non-volatile computer memory such as RAM, PROM, EPROM, and EEPROM. The memory unit can be used for storing program code that can be executed by a processor in the processing unit 52 to cause the signal analyzer 4 to perform the various functions and methods described herein.

In some embodiments, the signal analyzer 4 can comprise an interface or input 54 for receiving the modulated carrier signal 22 from the sensor circuit 10 via a wired connection (e.g. a wire that extends from a handle of a guide wire 6 where the signal analyzer 4 can be located) to the sensor circuit 10. In alternative embodiments where the modulated carrier signal 22 is communicated wirelessly from the sensor circuit 10, the signal analyzer 4 can comprise receiver circuitry 56 and associated antenna 58 for receiving the modulated carrier signal 22 from the sensor circuit 10.

Although not shown in Figure 6, the signal analyzer 4 can also receive RF field 14 and/or the pulsed field 20, or information on the RF field 14 (e.g. information on the frequency and/or phase of the RF field 14) and/or information on the pulses in the pulsed field 20 (e.g. information on the frequency and/or phase of the pulsed field 20), in which case the signal analyzer 4 can comprise an interface or input for receiving this information. Alternatively in embodiments where the signal analyzer 4 controls the generation of the pulsed field 20, the signal analyzer 4 will already have this information available.

As noted above, in some embodiments the signal analyzer 4 can also generate the RF field 14 and/or the pulsed field 20. Therefore the signal analyzer can comprise an RF field generator for generating the RF field 14 and/or a pulsed field generator for generating the pulsed field 20.

The signal analyzer 4 can then analyze the modulated carrier signal 22 to determine the measurement of the in-body property and/or the timing information relating to the modulated RF signal 22.

In some embodiments, the processing unit 52 analyses the modulated carrier signal 22 by comparing the amplitude, phase and/or frequency of the RF carrier signal 14 to the amplitude, phase and/or frequency of the received modulated carrier signal 22 to determine a measurement of the in-body property. The phase modulation of the carrier signal can be determined as a phase variation, or by comparing the phase to a reference signal, in this case the RF carrier signal 14.

In some embodiments, the processing unit 52 analyses the modulated carrier signal 22 by comparing the amplitude, phase and/or frequency of pulses in the pulsed signal 20 to the modulated carrier signal 22 to determine a timing of the modulated carrier signal 22 (where the timing is the delay due to travelling time through the body with the velocity of the ultrasound wave). For example the signal content of the phase and amplitude modulation can be compared with the signal content of the pulsed signal, to measure the delay in the modulation content with respect to the transmitted content.

In the processing set out above the processing unit 52 will analyze one or more sidebands of the modulated carrier signal 22 according to a phase of pulses in the pulsed field 20.

In embodiments where the sensor circuit 10 comprises a first transducer 16 and a third transducer 30 for measuring the in-body property at two different positions, with varactors 34, 36 (or other non-linear elements) for controlling which of the transducers 16, 30 modulates the RF carrier signal 14 in response to the phase of the pulsed field 20, the processing unit 52 is configured to analyze a first pair of sidebands of the modulated carrier signal 22 according to a first phase of pulses in the pulsed field 20 (e.g. a positive phase) to determine a measurement of the in-body property by the first transducer 16, and to analyze a second pair of sidebands of the modulated carrier signal 22 according to a second phase of pulses in the pulsed field 20 (e.g. a negative phase) to determine a measurement of the in-body property by the third transducer 30.

In some embodiments, the signal analyzer 4 can analyze the modulated carrier signal 22 to estimate the position of the sensor circuit 10 in the body. In one technique, it is possible to estimate the position of the sensor circuit 10 by determining a time-of-flight of the modulated carrier signal 22 using the timing information of the signal 22 (e.g. by comparing the timing of the pulses in the pulsed signal 20 to the timing of the modulation of the modulated carrier signal 22), and estimating the distance of the sensor circuit 10 from the receiver of the modulated signal 22 based on the time-of-flight and the propagation speed of the modulated signal (i.e. the speed of light). If the receiver of the modulated carrier signal 22 is able to determine the angle and/or direction from which the modulated carrier signal 22 was received (e.g. by using a directional receiver), then this angle and/or direction can be used with the distance estimate to estimate the position of the sensor circuit 10 relative to the receiver (e.g. relative to the signal analyzer 4).

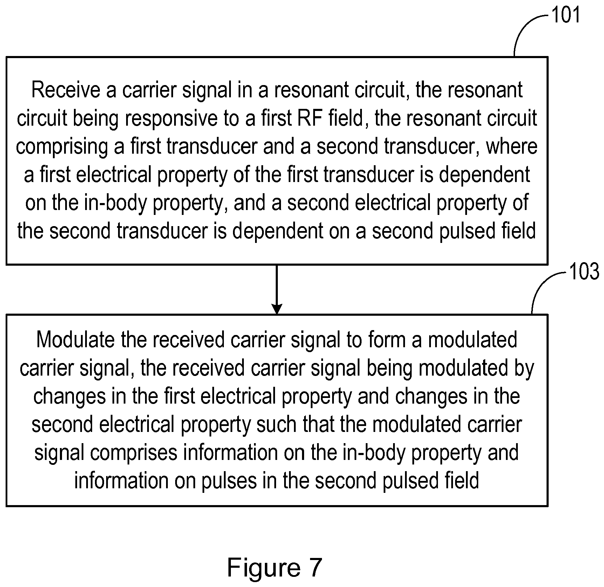

In an alternative technique, the modulated carrier signal 22 can be received by multiple receivers at different (but known) spatial positions, and the processing unit 52 can analyze each of the modulated carrier signals 22 to determine a respective time-of-flight measurement, and triangulation based on the time-of-flight measurements and known positions of the receivers can be used to estimate the position of the sensor circuit 10. Figure 7 is a flow chart illustrating a method of operating a sensor circuit 10 as described above to measure an in-body property according to the invention. The first step of the method, step 101, comprises receiving a carrier signal 14 in a resonant circuit 12. The resonant circuit 12 is responsive to a RF field 14 and comprises a first transducer 16 and a second transducer 18, with a first electrical property of the first transducer 16 being dependent on the in-body property, and a second electrical property of the second transducer 18 being dependent on a pulsed field 20.

In a second step, step 103, responsive to the pulsed field 20, the received carrier signal 14 is modulated to form a modulated carrier signal 22, with the received carrier signal 14 being modulated by changes in the first electrical property and changes in the second electrical property. In this wat the modulated carrier signal 22 will comprise information on the in-body property and information on pulses in the pulsed field 20 (e.g. information on the timing of the pulses).

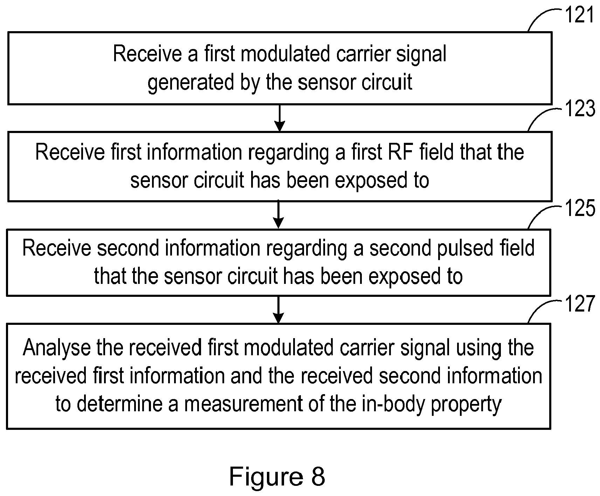

Figure 8 is a flow chart illustrating a method for measuring an in-body property using a sensor circuit 10 as described above according to the invention. This method can be implemented by signal analyzer 4, or more particularly by processing unit 52. In the first step of the method, step 121, the signal analyzer 4 receives a modulated carrier signal 22 generated by the sensor circuit 10. In step 123, the signal analyzer 4 receives information regarding an RF field 14 that the sensor circuit 10 has been exposed to, and in step 125 the signal analyzer 4 receives information regarding a pulsed field 20 that the sensor circuit 10 has been exposed to. Then, in step 127, the signal analyzer 4 analyses the modulated carrier signal 22 using the received information on the RF field 14 and the received information on the pulsed field 20 to determine a measurement of the in-body property.

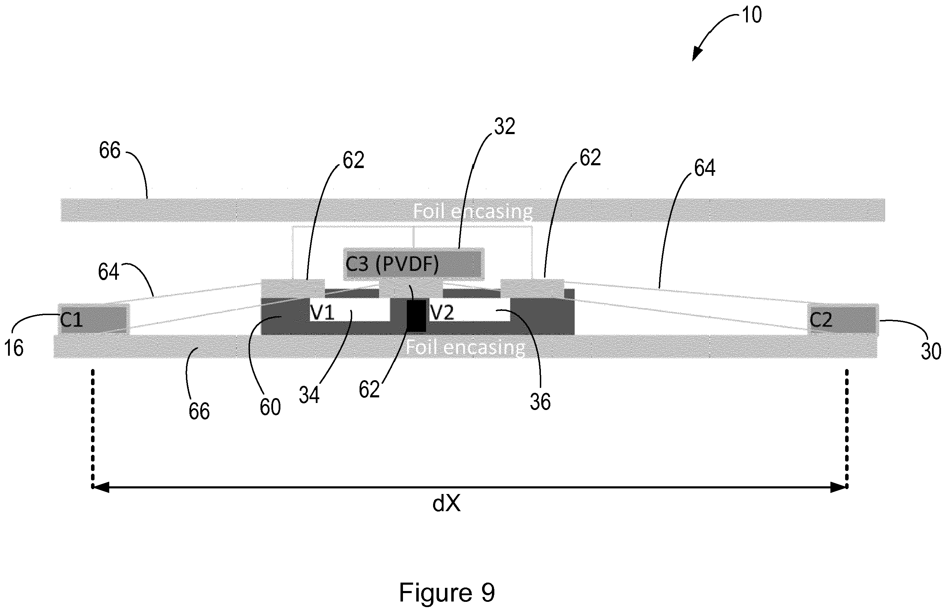

The sensor circuit 10 described above can be implemented on a catheter or guide wire 6, as all components of the sensor circuit 10 can be made with a thickness <25 µm and a sub-mm length and width, so adding very little to the overall diameter of a guide wire 6 or catheter, as well as adding little in the way of mechanical properties. Figure 9 shows an embodiment of the sensor circuit 10 in a wireless design. The two varactors 34, 36 could be made on one silicon slab 60 with three interconnect points 62 (e.g. formed from gold), the pvdf foil transducer 32 could be pressed on top of the middle interconnect point 62, and the air capacitors (the first transducer 16 (C₁) and the third transducer 30 (C₂)) connected using 10 µm gold or silver coated wires 64. A side view schematic of a sensor circuit 10 is given in Figure 9.

As the sensor circuit 10 in Figure 9 is a wireless design, an antenna of a few cm length is needed for pickup and reflection of the RF carrier wave 14. This embodiment can be used on non-electrically connected products such as catheters. In the example shown in Figure 9, the components are 3-10 µm thin, encased in 3-10 µm thin foil 66, interconnected by 10 µm diameter wires 64. The distance dX between C₁ 16 and C₂ 30 can be a few mms.

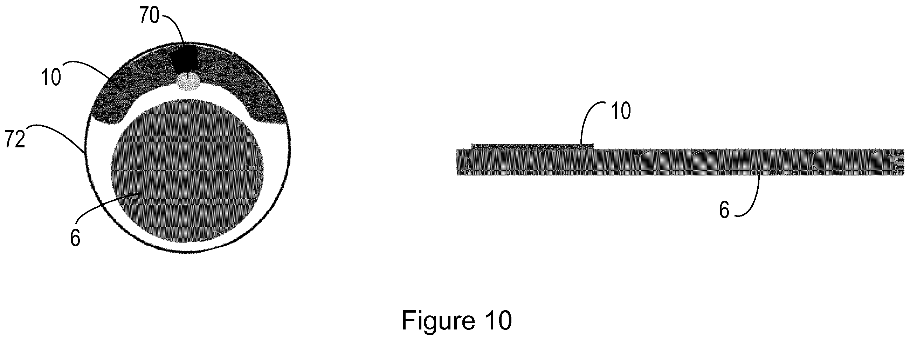

If the product on which the sensor circuit 10 is mounted is an electrically connected one, then it can be advantageous to use the electrical connection (wire) already present in the product, and interconnect by contacting the sensor circuit 10 with an exposed contact onto the product, as depicted in Figure 10. The sensor circuit 10 is placed on to a guidewire 6 with a contact 70 electrically connecting the sensor circuit 10 to the guide wire 6. The sensor circuit 10 can be mounted inside a shrink tube 72, and subsequently during shrinking pressed onto the product 6.

The invention can be used on various products, like RF receivers and localization, image fusion and flow measuring software. Furthermore, the invention can also be used as a stand-alone implantable, which, when the signal analyzer 4 determines a position of the sensor circuit 10 from the modulated carrier signal 22, could be used to guide a surgeon toward a previously identified target during an operation.

In some embodiments, a plurality of sensor circuits 10 could be provided on a guide wire 6 (or other product) that are to measure the in-body property at different locations in the body (including measuring the pressure flow at different locations). The resonant circuits 12 in each of the sensor circuits 10 can be tuned to respond to a respective RF frequency carrier signal, which means that measurements of the in-body property at a particular location on the guide wire 6 can be obtained by exposing the body to the RF carrier signal 14 corresponding to that sensor circuit 10. This enables pressure wave propagation speed measurements (or other in-body property measurements) on multiple parts of the guide wire 6. This can be particularly useful in FFR (fractional flow reserve) measurements, as no pull back of the wire 6 is needed when an occlusion in the blood vessel is passed, as there may already be a sensor circuit 10 on the wire 6 behind the occlusion.

Thus the solution of the present invention uses an RF field in combination with another pulsed external field to enable the communication of (a plurality of) sensor information to an external unit. The benefits of the second pulsed field are firstly to allow measurement timing determination of each of a plurality of sensors, and to allow improved RF modulation detection by lock in on the externally applied field to enable advanced computational interference decreasing methods to be used.

In this way, robust wireless time-framed measurement data transfer from in-body to an external unit is possible, enabling many in-body measurements (flow, analyte concentration etc.) without affecting the workflow.

The invention can be integrated in a range of guide wires 6 of different diameters, enabling also use with a variety catheters and workflows due to the absence of interconnects (in some embodiments) to be made.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A sensor circuit for measuring an in body property, the sensor circuit comprising:
a resonant circuit that is responsive to a first radio frequency, RF, field to receive a carrier signal, the resonant circuit having:
a first transducer, wherein a first electrical property of the first transducer is dependent on the in-body property, and
a second transducer, wherein a second electrical property of the second transducer is dependent on a second pulsed field;
wherein the carrier signal received by the resonant circuit is modulated by changes in the first electrical property due to the in-body property and changes in the second electrical property due to pulses in the second pulsed field.

2. A sensor circuit as claimed in claim 1, wherein the carrier signal received by the resonant circuit is modulated by changes in the first electrical property and changes in the second electrical property such that the modulated carrier signal comprises information on the in-body property and information on the pulses in the second pulsed field.

3. A sensor circuit as claimed in claim 1 or 2, wherein the second transducer comprises:
a first transducer component that generates a voltage responsive to the second pulsed field; and
a second transducer component coupled to the first transducer component, the second transducer component having the second electrical property, and wherein the second transducer component is such that the second electrical property is dependent on the voltage generated by the first transducer component.

4. A sensor circuit as claimed in claim 3, wherein the resonant circuit further comprises:
a third transducer, wherein a third electrical property of the third transducer is dependent on the in-body property, and wherein the third transducer is spaced from the first transducer such that the first transducer and the third transducer measure the in-body property at different positions in a body;
wherein the first transducer and the second transducer component form a first resonant sub-circuit; and
wherein the second transducer further comprises:
a third transducer component coupled to the first transducer component, and coupled to the third transducer to form a second resonant sub-circuit, the third transducer component having the second electrical property, and wherein the third transducer component is such that the second electrical property is dependent on the voltage generated by the first transducer component.

5. A sensor circuit as claimed in claim 4, wherein the second transducer component and the third transducer component are arranged such that the first transducer and the second transducer component generate at least one pair of sidebands for the modulated carrier signal during a first phase of the second pulsed signal, and such that the third transducer component and the third transducer generate at least one pair of sidebands for the modulated carrier signal during a second phase of the second pulsed signal, wherein the second phase is opposite to the first phase.

6. A sensor circuit as claimed in any of claims 1-5, wherein the in-body property is any one or more of pressure, temperature, conductivity, permeability and permittivity.

7. A sensor circuit as claimed in any of claims 1-6, wherein the second pulsed field is an ultrasound field, an X-ray field, an electromagnetic, EM, field used in magnetic resonance imaging, MRI, or light.

8. A signal analyzer for determining a measurement of an in-body property, the signal analyzer comprising:
a processing unit configured to:
receive a first modulated carrier signal generated by a sensor circuit;
receive first information regarding a first radio, RF, field that the sensor circuit has been exposed to;
receive second information regarding a second pulsed field that the sensor circuit has been exposed to; and
analyze the received first modulated carrier signal using the received first information and the received second information to determine a measurement of the in-body property.

9. A signal analyzer as claimed in claim 8, wherein the processing unit is configured to analyze the received first modulated carrier signal by:
comparing amplitude, phase and/or frequency of the first RF signal to the amplitude, phase and/or frequency of the received first modulated carrier signal to determine a measurement of the in-body property.

10. A signal analyzer as claimed in claim 8 or 9, wherein the processing unit is configured to analyze the received first modulated carrier signal by:
comparing amplitude, phase and/or frequency of pulses in the second pulsed signal to the received first modulated carrier signal to determine a timing of the modulated carrier signal.

11. A signal analyzer as claimed in claim 10, wherein the processing unit is configured to:
analyze a first pair of sidebands of the received first modulated carrier signal according to a first phase of pulses in the second pulsed field to determine a measurement of the in-body property by a first transducer, wherein a first electrical property of the first transducer is dependent on the in-body property; and
analyze a second pair of sidebands of the received first modulated carrier signal according to a second phase of pulses in the second pulsed field to determine a measurement of the in-body property by a second transducer, wherein the second phase is opposite to the first phase and wherein a second electrical property of the second transducer is dependent on the in-body property.

12. A system for measuring an in-body property comprising:
a sensor circuit as claimed in any of claims 1-7; and
a signal analyzer as claimed in any of claims 8-11.

13. A system as claimed in claim 12, wherein the system further comprises one or both of:
a first field generator for generating the first RF field; and
a second pulsed field generator for generating the second pulsed field.

14. A method of operating a sensor circuit to measure an in-body property, the method comprising:
receiving a carrier signal in a resonant circuit, the resonant circuit being responsive to a first radio frequency, RF, field, the resonant circuit comprising a first transducer and a second transducer, wherein a first electrical property of the first transducer is dependent on the in-body property, and a second electrical property of the second transducer is dependent on a second pulsed field;
responsive to the second pulsed field, modulating the received carrier signal to form a modulated carrier signal, wherein the received carrier signal is modulated by changes in the first electrical property and changes in the second electrical property such that the modulated carrier signal comprises information on the in-body property and information on pulses in the second pulsed field.

15. A method for measuring an in-body property using a sensor circuit, the method comprising:
receiving a first modulated carrier signal generated by the sensor circuit;
receiving first information regarding a first radio frequency, RF, field that the sensor circuit has been exposed to;
receiving second information regarding a second pulsed field that the sensor circuit has been exposed to; and
analyzing the received first modulated carrier signal using the received first information and the received second information to determine a measurement of the in-body property.
